(19) Europäisches Patentamt
European Patent Office
Office européen des brevets

(11) Veröffentlichungsnummer: **0 132 568**
**B1**

(12) **EUROPÄISCHE PATENTSCHRIFT**

(45) Veröffentlichungstag der Patentschrift: **28.01.87**

(51) Int. Cl.[4]: **C 07 C 91/06**, A 61 K 7/13

(21) Anmeldenummer: **84106791.1**

(22) Anmeldetag: **14.06.84**

(54) **1,4-Diamino-5-chlor-2-nitrobenzolderivate, Verfahren zu ihrer Herstellung und Mittel zur Färbung von Haaren.**

(30) Priorität: **28.06.83 DE 3323207**

(43) Veröffentlichungstag der Anmeldung: **13.02.85 Patentblatt 85/7**

(45) Bekanntmachung des Hinweises auf die Patenterteilung: **28.01.87 Patentblatt 87/5**

(84) Benannte Vertragsstaaten: **AT BE CH DE FR GB IT LI LU NL SE**

(56) Entgegenhaltungen:
**DE - A - 1 569 814**
**FR - A - 2 135 138**
**FR - A - 2 348 911**

**Die Akte enthält technische Angaben, die nach dem Eingang der Anmeldung eingereicht wurden und die nicht in dieser Patentschrift enthalten sind.**

(73) Patentinhaber: **Wella Aktiengesellschaft, Berliner Allee 65, D-6100 Darmstadt (DE)**

(72) Erfinder: **Konrad, Eugen, Mecklenburger Strasse 101, D-6100 Darmstadt (DE)**
Erfinder: **Clausen, Thomas, Dr., Rheinstrasse 19, D-6108 Weiterstadt (DE)**

**EP 0 132 568 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung sind Mittel zur Färbung von Haaren mit Nitrofarbstoffen, wobei als Nitrofarbstoffe neue Derivate des 1,4-Diamino-5-chlor-2--nitrobenzols verwendet werden sowie ein Verfahren zur Herstellung dieser Derivate.

Für die Haarfärbung haben Nitrofarbstoffe eine wesentliche Bedeutung erlangt. Durch Kombination verschiedener Nitrofarbstoffe lassen sich Färbemittel herstellen, die ohne Zusatz von Oxidationsmitteln Haare in natürlichen und modischen Tönen zu färben vermögen. Die Nitrofarbstoffe sind ebenfalls wichtige Bestandteile von Oxidationshaarfärbemitteln, da sie es auf einfache Art ermöglichen, natürliche oder modische Farbnuancen herzustellen.

An Nitrofarbstoffe, die zum Färben von menschlichen Haaren Verwendung finden, sind zahlreiche besondere Anforderungen gestellt. So müssen sie in toxikologischer und dermatologischer Hinsicht unbedenklich sein und die Erzielung von Färbungen in der gewünschten Intensität ermöglichen, was unter anderem auch eine ausreichende Wasserlöslichkeit voraussetzt. Ausserdem wird für die erzielten Haarfärbungen eine gute Licht-, Säure- und Reibechtheit gefordert. Ihre Verwendung in Oxidationshaarfärbemitteln setzt weiterhin voraus, dass sie in Anwesenheit von Hydrogenperoxid in ammoniakalischer Lösung stabil sind.

Zur Zeit werden in Haarfärbemitteln als Nitrofarbstoffe, neben anderen Farbstoffen, Derivate des o- und p-Phenylendiamins eingesetzt, z.B. der gelbe Farbstoff 4-Nitro-o-phenylendiamin oder das rote 2-Nitro-p-phenylendiamin. Diese Farbstoffe erfüllen aber die oben genannten Bedingungen, insbesondere die physiologischen Voraussetzungen, nur ungenügend.

In der FR-A-2 348 911 sind Haarfärbemittel auf der Basis von 3-Nitro-4-(β-hydroxyethyl)-amino--phenolverbindungen beschrieben, welche zusätzlich unsubstituierte oder substituierte Amino-Nitrobenzolfarbstoffe einer allgemeinen Formel enthalten können. Diese allgemeine Formel umfasst eine sehr grosse Anzahl von Verbindungen, darunter auch solche mit Halogen- und Hydroxyalkylamino-Substituenten. Verbindungen mit Hydroxypropyl- oder Dihydroxypropyl-Substituenten sind dort nicht genannt.

Aus der DE-A-2 157 844 (= FR-A-2 135 138) sind weiterhin 5-Chlor-2-nitro-p-phenylendiaminderivate bekannt, die diese Voraussetzungen besser erfüllen. Ein Nachteil der dort offenbarten Verbindungen ist aber ihre relativ geringe Wasserlöslichkeit, welche den Einsatz in höherer Konzentration zur Erzielung grösserer Farbtiefen verhindert.

Es wurde nun gefunden, dass sich dieser Nachteil beseitigen lässt durch Verwendung von Haarfärbemitteln mit einem Gehalt an 1,4-Diamino-5-chlor-2--nitrobenzolderivaten der allgemeinen Formel I

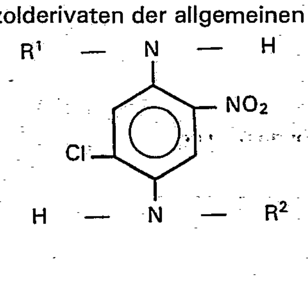

(I),

worin $R^1$ und $R^2$ gleich oder verschieden sein können und Wasserstoff, 2-Hydroxyethyl oder 2,3-Dihydroxypropyl bedeuten, mit der Massgabe, dass mindestens einer der Reste $R^1$ oder $R^2$ 2,3-Dihydroxypropyl darstellt.

Diese Farbstoffe sind neu und ergeben Haarfärbungen in reinen roten bis violetten Farbtönen von hervorragender Stabilität. Ihre Herstellung, die in der Anmeldung näher beschrieben ist, kann nach zwei verschiedenen Wegen erfolgen. So können die Verbindungen der Formel I einerseits ausgehend vom 1,4-Diamino-5-chlor-2-nitrobenzol, das gegebenenfalls an einer der beiden Aminogruppen mit einer 2-Hydroxyethylgruppe substituiert sein kann, durch Umsetzung mit Chlorpropandiol-(2,3) in alkalischem Medium erhalten werden. Im anderen Falle wird, ausgehend vom 5-Acetylamino-2,4-dichlor-nitrobenzol, ein Chloratom mit 1-Amino-propandiol-(2,3) nucleophil gegen die Aminogruppe ausgetauscht und danach die Acetylgruppe verseift, wobei die freie Aminogruppe gegebenenfalls anschliessend noch ethoxyliert werden kann. Die Herstellung der Ausgangsmaterialien, nämlich des 1,4-Diamino-5-chlor-2--nitrobenzols, der hydroxyethylierten 1,4-Diamino--5-chlor-2-nitrobenzolderivate und des 5-Acetylamino-2,4-dichlornitrobenzols, ist bekannt und unter anderem in der bereits vorstehend genannten DE-A-2 157 844 beschrieben.

Die erfindungsgemässen Farbstoffe der Formel I sind besser wasserlöslich als die Farbstoffe nach der DE-A-2 157 844. Überraschenderweise zeigen sie zudem auch in toxikologischer Hinsicht und im Amestest bezüglich der mutagenen Wirkung günstigere Eigenschaften als die Farbstoffe gemäss der oben genannten DE-A-2 157 844.

Die erfindungsgemässen Mittel zur Färbung von Haaren betreffen sowohl solche, die ohne Zugabe eines Oxidationsmittels angewendet werden, als auch solche, bei denen die Zugabe eines Oxidationsmittels erforderlich ist.

Bei den ersteren Haarfärbemitteln ohne Oxidationsmittel-Zugabe handelt es sich um diejenigen, die neben den Farbstoffen der angegebenen Formel noch andere direkt auf das Haar aufziehende Farbstoffe enthalten können. Von diesen für die Haarfärbung bekannten Farbstoffen seien beispielsweise die folgenden Klassen erwähnt:

Aromatische Nitrofarbstoffe (z.B. 1,2-Diamino-4--nitrobenzol), Azofarbstoffe (z.B. Acid Brown 4, C.I. 14 805), Anthrachinonfarbstoffe (z.B. Disperse Violet 4, C.I. 61 105), Triphenylmethanfarbstoffe (z.B. Basic Violet 1, C.I. 61 100), wobei die Farbstoffe dieser Klassen je nach der Art ihrer Substituenten sauren, nichtionogenen oder basischen Charakter haben können. Weitere geeignete direkt auf das Haar aufziehende Farbstoffe sind beispielsweise in dem Buch von J.C. Johnson, «Hair Dyes» Noyes Data Corp. Park-Ridge (USA) (1973) beschrieben.

Mit Haarfärbemitteln, die derartige Gemische von Farbstoffen enthalten, lassen sich neben reinen Modetönen auch modische Blond- und Brauntöne von hervorragender Stabilität erzielen.

Die Zubereitungsform der hier beschriebenen Haarfärbemittel auf der Basis von direkt auf das Haar aufziehenden Farbstoffen kann beispielsweise eine

Lösung, insbesondere eine wässrige oder wässrig-alkoholische Lösung sein. Bevorzugte Zubereitungsformen sind weiterhin eine Creme, ein Gel oder eine Emulsion, wobei sie auch im Gemisch mit einem Treibgas oder mittels einer Pumpe versprüht werden können.

Die Farbstoffe der angegebenen Formel I sollen in diesen Färbemitteln in einer Konzentration von etwa 0,01 bis 2,0 Gew.-%, vorzugsweise von 0,01 bis 1,0 Gew.-%, enthalten sein. Der Gesamtgehalt an Farbstoffen liegt in den Grenzen von etwa 0,01 bis 3,0 Gew.-%.

Der pH-Wert dieser Färbemittel liegt im Bereich von 7 bis 10,5, insbesondere bei pH 7,5 bis 9,5, wobei die Einstellung des gewünschten pH-Wertes hauptsächlich mit Ammoniak erfolgt, jedoch auch mit organischen Aminen wie beispielsweise Monoethanolamin oder Triethanolamin vorgenommen werden kann.

Ihre Anwendung erfolgt in üblicher Weise durch Aufbringen des Mittels auf die Haare, mit denen es eine Zeitlang, zwischen 5 und 30 Minuten, in Berührung bleibt. Anschliessend wird mit Wasser, gegebenenfalls noch mit einer schwachen organischen Säure, gespült und getrocknet. Als schwache organische Säuren können beispielsweise Essigsäure, Zitronensäure, Weinsäure und ähnliche Verwendung finden.

Die vorstehend beschriebenen Haarfärbemittel ohne Oxidationsmittel-Zugabe können selbstverständlich auch kosmetische Polymerisate enthalten, wodurch gleichzeitig mit der Färbung eine Festigung der Haare erreicht wird. Solche Mittel werden im allgemeinen als Tönungsfestiger oder Farbfestiger bezeichnet.

Von den für diesen Zweck in der Kosmetik bekannten Polymerisaten seien beispielsweise Polyvinylpyrrolidon, Polyvinylacetat, Polyvinylalkohol oder Polyacrylverbindungen wie Acrylsäure- beziehungsweise Methacrylsäurepolymerisate, basische Polymerisate von Estern aus diesen beiden Säuren und Aminoalkoholen beziehungsweise deren Salze oder Quaternisierungsprodukte, Polyacrylnitril, Polyvinyllactame sowie Copolymerisate aus derartigen Verbindungen wie Polyvinylpyrrolidon-Vinylacetat und dergleichen erwähnt.

Auch natürliche Polymere, wie Chitosan (entacetyliertes Chitin) oder Chitosanderivate, können für den genannten Zweck eingesetzt werden.

Die Polymerisate sind in diesen Mitteln in der üblichen Menge von etwa 1 bis 4 Gew.-% enthalten. Die pH-Werte der Mittel liegen im Bereich von etwa 6,0 bis 9,0.

Die Anwendung dieser Haarfärbemittel mit zusätzlicher Festigung erfolgt in bekannter und üblicher Weise durch Befeuchten des Haares mit dem Festiger, Festlegen (Einlegen) des Haares zur Frisur und anschliessende Trocknung.

Selbstverständlich können die vorstehend beschriebenen Haarfärbemittel ohne Oxidationsmittel-Zugabe gegebenenfalls weitere gebräuchliche kosmetische Zusätze wie z.B. Pflegestoffe, Netzmittel, Verdicker, Weichmacher und Parfümöle enthalten.

Zum Gegenstand der vorliegenden Erfindung gehören auch, wie eingangs erwähnt, diejenigen Haarfärbemittel, bei denen die Zugabe eines Oxidationsmittels erforderlich ist. Sie enthalten ausser den Farbstoffen gemäss angegebener Formel noch zusätzlich bekannte Oxidationsfarbstoffe, die einer oxidativen Entwicklung bedürfen.

Bei diesen Oxidationsfarbstoffen handelt es sich hauptsächlich um aromatische p-Diamine und p-Aminophenole wie beispielsweise p-Toluylendiamin, p-Phenylendiamin, p-Aminophenol und ähnliche Verbindungen, welche zum Zwecke der Nuancierung der Färbungen mit sogenannten Modifiern, wie z.B. m-Phenylendiamin, Resorcin, m-Aminophenol und anderen kombiniert werden.

Derartige zur Haarfärbung bekannte und übliche Oxidationsfarbstoffe sind unter anderem in dem Buch von E. Sagarin, «Cosmetics», Science and Technology (1957), Interscience Publishers Inc., New York, Seiten 503 ff. sowie in dem Buch von H. Janistyn, «Handbuch der Kosmetika und Riechstoffe» (1973) Seiten 338 ff., beschrieben.

Mit Mischungen aus diesen Oxidationsfarbstoffen und den Farbstoffen gemäss der angegebenen Formel lassen sich neben reinen Modetönen auch modische Blond- und Brauntöne erhalten.

Die Farbstoffe gemäss der Formel I sind in diesen Färbemitteln mit Oxidationsmittel-Zugabe in einer Konzentration von etwa 0,01 bis 2,0 Gew.-%, vorzugsweise 0,05 bis 1,0 Gew.-%, enthalten. Der Gesamtgehalt an Farbstoffen in diesen Färbemitteln beträgt etwa 0,1 bis 5,0 Gew.-%.

Oxidationshaarfärbemittel sind im allgemeinen alkalisch eingestellt, vorzugsweise auf pH-Werte von etwa 8,0 bis 11,5, wobei die Einstellung insbesondere mit Ammoniak erfolgt. Es können dazu aber auch andere organische Amine, z.B. Monoethanolamin oder Triethanolamin, Verwendung finden. Als Oxidationsmittel zur Entwicklung der Haarfärbungen kommen hauptsächlich Hydrogenperoxyd und dessen Additionsverbindungen in Betracht. Die Zubereitungsform dieser Haarfärbemittel kann die gleiche wie bei Haarfärbemitteln ohne Oxidationsmittelzugabe sein. Vorzugsweise ist sie die einer Creme oder eines Gels.

Übliche Zusätze in Cremes, Emulsionen oder Gelen sind z.B. Lösungsmittel wie Wasser, niedere aliphatische Alkohole, beispielsweise Ethanol, Propanol, Isopropanol, Glycerin oder Glykole wie Ethylenglykol und Propylenglykol, oder auch Glykolether, weiterhin Netzmittel oder Emulgatoren aus den Klassen der anionischen, kationischen, amphoteren oder nichtionogenen oberflächenaktiven Substanzen wie Fettalkoholsulfate, Fettalkoholethersulfate, Alkylsulfonate, Alkylbenzolsulfate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Bentonit, Stärke, Polyacrylsäure, Cellulosederivate, wie Carboxymethylcellulose, Alginate, Vaseline, Paraffinöl und Fettsäuren sowie ausserdem Pflegestoffe wie Lanolinderivate, Cholesterin, Pantothensäure und Betain, weiterhin Parfümöle und Komplexbildner. Die erwähnten Bestandteile werden in den für solche Zwecke üblichen Mengen verwendet, z.B. die Netzmittel und Emulgatoren in Konzentrationen von etwa 0,5 bis 30 Gew.-%, wäh-

rend die Verdicker in einer Menge von etwa 0,1 bis 25 Gew.-% in den Zubereitungen enthalten sein können.

Die Anwendung der genannten Zubereitungen erfolgt in bekannter Weise, indem man die Haarfärbemittel vor der Behandlung mit dem Oxidationsmittel vermischt und die Mischung auf das Haar aufträgt.

Nach einer für die Haarfärbung ausreichenden Einwirkungszeit, welche üblicherweise etwa 10 bis 45 Minuten beträgt, wird mit Wasser, gegebenenfalls anschliessend mit einer schwachen organischen Säure, wie z.B. Zitronensäure oder Weinsäure, gespült und getrocknet.

Die nachstehenden Beispiele sollen den Anmeldungsgegenstand erläutern, ohne ihn darauf zu beschränken.

*Beispiele für kosmetische Mittel*

*Beispiel 1*

Das flüssige Haarfärbemittel der Zusammensetzung

| | |
|---|---|
| 0,2 g | 1-Amino-4-(2′,3′-dihydroxypropyl)-amino-5-chlor-2-nitrobenzol |
| 0,5 g | Hydroxyethylcelulose |
| 5,0 g | Laurylalkohol-diglykolethersulfat-Natriumsalz, 28%ige wässrige Lösung |
| 15,0 g | Isopropylalkohol |
| 0,03 g | Ammoniak, 25%ig |
| 79,27 g | Wasser |
| 100,00 g | |

wird auf weisse menschliche Haare aufgetragen und 10 Minuten einwirken gelassen. Nach Spülung mit Wasser und Trocknung ist das Haar leuchtendrot gefärbt.

*Beispiel 2*

Farbfestiger

| | |
|---|---|
| 0,10 g | 1-Amino-4-(2′,3′-dihydroxypropyl)-amino-5-chlor-2-nitrobenzol |
| 2,00 g | Polyvinylpyrrolidon |
| 0,10 g | Glycerin |
| 40,00 g | Isopropylalkohol |
| 57,80 g | Wasser |
| 100,00 g | |

Weisse menschliche Haare werden mit der festigenden Färbelösung zur Frisur eingelegt und getrocknet. Das Haar ist leuchtendrot gefärbt und gefestigt.

*Beispiel 3*

Oxidationshaarfärbemittel

| | |
|---|---|
| 0,2 g | 4-Amino-1-(2′,3′-dihydroxypropyl)-amino-5-chlor-2-nitrobenzol |
| 35,0 g | Ölsäure |
| 15,0 g | Isopropylalkohol |
| 18,0 g | Ammoniak, 25%ig |
| 0,2 g | Dinatriumsalz der Ethylendiamin-tetraessigsäure |
| 0,1 g | Natriumsulfit |
| 0,8 g | p-Toluylendiamin-sulfat |
| 0,2 g | Resorcin |
| 0,05 g | m-Aminophenol |
| 30,45 g | Wasser |
| 100,00 g | |

50 ml des vorstehenden Haarfärbemittels werden kurz vor dem Gebrauch mit 50 ml Hydrogenperoxydlösung (6%ig) gemischt. Das entstandene Gel wird anschliessend auf graue menschliche Haare aufgetragen und 30 Minuten einwirken gelassen. Sodann wird mit Wasser gespült und getrocknet. Das Haar hat eine rötlichblonde Färbung erhalten.

*Beispiel 4*

Farbfestiger

| | |
|---|---|
| 0,15 g | 4-Amino-1-(2′,3′-dihydroxypropyl)-amino-5-chlor-2-nitrobenzol |
| 2,0 g | Copolymerisat von Vinylpyrrolidon-vinylacetat 60:40 |
| 0,1 g | Glycerin |
| 40,0 g | Isopropylalkohol |
| 57,75 g | Wasser |
| 100,00 g | |

Man legt weisse menschlische Haare mit dieser festigenden Färbelösung zur Frisur ein und trocknet anschliessend. Die Haare sind bläulichrot gefärbt und gefestigt.

*Beispiel 5*

Ein flüssiges Haarfärbemittel, bestehend aus

| | |
|---|---|
| 0,1 g | 4-Amino-1-(2′,3′-dihydroxypropyl)-amino-5-chlor-2-nitrobenzol |
| 5,0 g | Laurylalkohol-diglykolethersulfat-Natriumsalz, 28%ige wässrige Lösung |
| 0,5 g | Hydroxyethylcellulose |
| 15,0 g | Ethylalkohol |
| 0,03 g | Ammoniak, 25%ig |
| 79,37 g | Wasser |
| 100,00 g | |

wird auf weisse menschliche Haare aufgebracht und 10 Minuten einwirken gelassen. Sodann wird mit Wasser gespült und getrocknet. Das Haar hat eine bläulichrote Färbung erhalten.

*Beispiel 6*

Ein flüssiges Haarfärbemittel der Zusammensetzung

| | |
|---|---|
| 0,1 g | 4-Amino-1-(2′,3′-dihydroxypropyl)-amino-5-chlor-2-nitrobenzol |
| 0,5 g | Hydroxyethylcellulose |
| 5,0 g | Laurylalkohol-diglykolethersulfat-Natriumsalz, 28%ige wässrige Lösung |

| | |
|---|---|
| 15,0 g | Isopropylalkohol |
| 0,3 g | Acid Brown 4 (C.I. 14 805) |
| 0,03 g | Ammoniak, 25%ig |
| 79,07 g | Wasser |
| 100,00 g | |

wird auf weisse menschliche Haare aufgetragen und 10 Minuten lang einwirken gelassen. Nach Spülung mit Wasser und Trocknung ist das Haar rotbraun gefärbt.

*Beispiel 7*

Ein flüssiges Haarfärbemittel der Zusammensetzung

| | |
|---|---|
| 0,1 g | 1-(2'-Hydroxyethyl)-amino-4-(2',3'-dihydroxypropyl)-amino-5-chlor-2-nitrobenzol |
| 0,5 g | Hydroxyethylcellulose |
| 5,0 g | Laurylalkohol-diglykolethersulfat-Natriumsalz, 28%ige wässrige Lösung |
| 15,0 g | Isopropylalkohol |
| 0,03 g | Ammoniak, 25%ig |
| 79,37 g | Wasser |
| 100,00 g | |

wird auf weisse menschliche Haare aufgetragen und 10 Minuten einwirken gelassen. Nach Spülung mit Wasser und Trocknung ist das Haar violett gefärbt.

*Beispiel 8*

Farbfestiger

| | |
|---|---|
| 0,15 g | 1-(2'-Hydroxyethyl)-amino-4-(2',3'-dihydroxypropyl)-amino-5-chlor-2-nitrobenzol |
| 2,00 g | Polyvinylpyrrolidon |
| 0,10 g | Glycerin |
| 40,00 g | Isopropylalkohol |
| 57,75 g | Wasser |
| 100,00 g | |

Weisse menschliche Haare werden mit der vorstehend festigenden Färbelösung zur Frisur eingelegt und getrocknet. Das Haar ist violett gefärbt und gefestigt.

*Beispiel 9*

Oxidationshaarfärbemittel

| | |
|---|---|
| 0,2 g | 1,4-Di-[(2',3'-dihydroxypropyl)-amino]-5-chlor-2-nitrobenzol |
| 35,0 g | Ölsäure |
| 15,0 g | Isopropylalkohol |
| 18,0 g | Ammoniak, 25%ig |
| 0,2 g | Dinatriumsalz der Ethylendiamin-tetraessigsäure |
| 0,1 g | Natriumsulfit |
| 0,8 g | p-Toluylendiamin-sulfat |
| 0,2 g | Resorcin |
| 0,05 g | m-Aminophenol |
| 30,45 g | Wasser |
| 100,00 g | |

50 ml des vorstehenden Haarfärbemittels werden kurz vor dem Gebrauch mit 50 ml Hydrogenperoxydlösung (6%ig) gemischt. Das entstandene Gel wird anschliessend auf graue menschliche Haare aufgetragen und 30 Minuten einwirken gelassen. Sodann wird mit Wasser gespült und getrocknet. Das Haar hat eine rötlichblonde Färbung erhalten.

*Beispiel 10*

Das flüssige Haarfärbemittel der Zusammensetzung

| | |
|---|---|
| 0,7 g | 1-Amino-4-(2',3'-dihydroxypropyl)-amino-5-chlor-2-nitrobenzol |
| 0,3 g | 1,4-Di-[(2',3'-dihydroxypropyl)-amino]-5-chlor-2-nitrobenzol |
| 0,5 g | Hydroxyethylcellulose |
| 5,0 g | Laurylalkohol-diglykolethersulfat-Natriumsalz, 28%ige wässrige Lösung |
| 15,0 g | Isopropylalkohol |
| 0,05 g | Ammoniak, 25%ig |
| 78,45 g | Wasser |
| 100,00 g | |

wird auf weisse menschliche Haare aufgetragen und 15 Minuten einwirken gelassen. Nach Spülung mit Wasser und Trocknung ist das Haar dunkelrotviolett eingefärbt.

*Herstellungsbeispiele*

*Beispiel 11*

1-Amino-4-(2',3'-dihydroxypropyl)-amino-5-chlor-2-nitrobenzol

18,7 g (0,1 Mol) 1,4-Diamino-5-chlor-2-nitrobenzol werdenin 40 ml Dimethylformamid gelöst und mit 44,0 g Chlorpropandiol-(2,3) versetzt. Man erhitzt auf 150°C (Ölbadtemperatur) und lässt eine Lösung von 20,0 g Natriumhydroxid in 200 ml Wasser innerhalb von $2^1/_2$ Stunden zutropfen. Nach dem Abkühlen wird mit Methylenchlorid extrahiert, wobei das Dimethylformamid und überschüssiges Chlorpropandiol als Lösungsvermittler für die Dihydroxypropylverbindung wirken. Das Methylenchlorid wird im Vakuum verdampft. Bei der darauffolgenden Zugabe von Wasser zum Rückstand kristallisiert das 1-Amino-4-(2',3'-dihydroxypropyl)-amino-5-chlor-2-nitrobenzol aus. Nach Umkristallisation aus Wasser schmilzt die Verbindung bei 126°C. Ausbeute: 5,0 g.

*Infrarot-Absorptionsbanden ($cm^{-1}$):*

| | | |
|---|---|---|
| 3000 - 3600 (breit) | 1414 | 1020 |
| 1563 | 1327 | 995 |
| 1520 | 1222 (breit) | 860 |
| 1480 | 1115 | 760 |

*Beispiel 12*

1-Amino-4-(2',3'-dihydroxypropyl)-amino-5-chlor-2-nitrobenzol

2,49 g (0,01 Mol) 2-Nitro-4-acetylamino-1,5-dichlorbenzol werden in 18 ml 2-Methoxy-ethanol gelöst und mit 5,4 g (0,06 Mol) 1-Aminopropandiol-(2,3) 4 Stunden auf 150°C (Ölbadtemperatur) erwärmt. Nach dem Abkühlen fällt bei längerem Stehenlassen die Acetylverbindung aus. Man saugt ab, verseift mit alkoholischer Salzsäure und fällt die Base mit Ammoniak aus. Die aus Essigester umkristallisierte Verbindung schmilzt bei 162°C. Ausbeute: 0,5 g.

*Infrarot-Absorptionsbanden (cm$^{-1}$):*

| | | |
|---|---|---|
| 3000 - 3600 (breit) | 1510 sh | 1195 |
| 1625 | 1411 | 1024 |
| 1562 | 1314 | 998 |
| 1515 | 1240 | |

*Beispiel 13*

1-(2'-Hydroxyethyl)-amino-4-(2',3'-dihydroxypropyl)-amino-5-chlor-2-nitrobenzol

2,3 g (0,01 Mol) 1-(2'-Hydroxyethyl)-amino-4-amino-5-chlor-2-nitrobenzol werden mit 5 ml $H_2O$ und 5,5 g Chlorpropandiol-(2,3) (0,05 Mol) auf 130°C (Ölbadtemperatur) erwärmt. Man lässt 1,25 g Natriumhydroxid in 12,5 ml $H_2O$ zutropfen. Nach 30 Minuten ist die Reaktion beendet. Beim Abkühlen fällt die Substanz aus. Man kristallisiert aus Wasser um und erhält 1,2 g der gesuchten Verbindung vom Schmelzpunkt 172°C.

*Infrarot-Absortionsbanden (cm$^{-1}$):*

| | | |
|---|---|---|
| 3000 - 3600 (breit) | 1335 | 1000 |
| 1565 | 1195 (breit) | 860 |
| 1525 | 1048 | 755 |
| 1405 | 1025 | |

*Beispiel 14*

1,4-Di[(2',3'-dihydroxypropyl)-amino]-5-chlor-2-nitrobenzol

18,7 g (0,1 Mol) 1,4-Diamino-5-chlor-2-nitrobenzol und 22 g (0,2 Mol) 3-Chlorpropandiol-(1,2) werden in 20 ml Wasser auf 150°C (Ölbadtemperatur) erwärmt. Man lässt unter Rühren eine Lösung von 8 g Natriumhydroxid in 80 ml Wasser während 1 Stunde zutropfen und hält die Reaktionsmischung noch 1 Stunde bei 150°C. Man verdünnt dann mit Wasser; beim Erkalten fällt ein Gemisch aus 1-Amino-4-(2',3'-dihydroxypropyl)-amino-5-chlor-2-nitrobenzol und 1,4-Di[(2',3'-dihydroxypropyl)-amino]-5-chlor-2-nitrobenzol aus. Man saugt ab und trocknet den Niederschlag. Ausbeute: 14 g eines etwa 1:1-Gemisches beider Verbindungen.

Zur Trennung wird aus 220 ml n-Butanol umkristalisiert. Das 1,4-Di-[(2',3'-dihydroxypropyl)-amino-5-chlor-2-nitrobenzol kristallisiert aus, während die Monoalkylverbindung in Lösung bleibt. Nochmalige Umkristallisation liefert die reine Dialkylverbindung in Kristallen vom Schmelzpunkt 208°C.

*Infrarot-Absorptionsbanden (cm$^{-1}$):*

| | | |
|---|---|---|
| 3000 - 3600 (breit) | 1330 | 1020 |
| 1635 | 1230 | 995 |
| 1565 | 1200 | 862 |
| 1525 | 1110 | 760 |
| 1405 | 1075 | |

Die Monoalkylverbindung kann durch Abdestillieren des n-Butanols und mehrmaliges Umkristallisieren aus Wasser ebenfalls rein gewonnen werden.

Alle in der vorliegenden Anmeldung angegebenen Prozentzahlen stellen Gewichtsprozente dar.

**Patentansprüche**

1. 1,4-Diamino-5-chlor-2-nitrobenzolderivate der allgemeinen Formel I

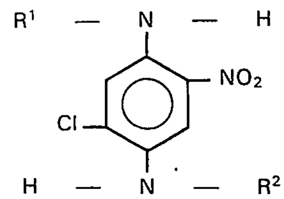

(I),

worin $R^1$ und $R^2$ gleich oder verschieden sein können und Wasserstoff, 2-Hydroxyethyl oder 2,3-Dihydroxypropyl bedeuten, mit der Massgabe, dass mindestens einer der Reste $R^1$ oder $R^2$ 2,3-Dihydroxypropyl darstellt.

2. 1-Amino-4-(2',3'-dihydroxypropyl)-amino-5-chlor-2-nitrobenzol.

3. 4-Amino-1-(2',3'-dihydroxypropyl)-amino-5-chlor-2-nitrobenzol.

4. 1-(2'-Hydroxyethyl)-amino-4-(2',3'-dihydroxypropyl)-amino-5-chlor-2-nitrobenzol.

5. 1,4-Di-[(2',3'-dihydroxypropyl)-amino]-5-chlor-2-nitrobenzol.

6. Mittel zur Färbung von Haaren mit einem Farbstoffgehalt und üblichen kosmetischen Zusätzen, dadurch gekennzeichnet, dass es mindestens eine Verbindung nach Anspruch 1 enthält.

7. Mittel nach Anspruch 6 mit einem pH-Wert von 8,0 bis 11,5, der durch Ammoniak oder organische Amine, wie Monoethanolamin oder Triethanolamin, eingestellt wurde.

8. Mittel zur oxidativen Färbung von Haaren nach Anspruch 6 und 7, zusätzlich enthaltend mindestens einen der bekannten Oxidationshaarfarbstoffe.

9. Mittel in Form eines Haarfärbemittels mit zusätzlicher Haarfestigung nach Anspruch 6, zusätzlich enthaltend in wässrig-alkoholischer Lösung bekannte direkt auf das Haar aufziehende Farbstoffe und mindestens ein übliches kosmetisches Polymerisat.

10. Verfahren zur Herstellung der Verbindung gemäss Anspruch 1, dadurch gekennzeichnet, dass man 1,4-Diamino-5-chlor-2-nitrobenzol, das gegebenenfalls an einer der Aminogruppen mit einer 2-Hydroxyethylgruppe substituiert ist, mit Chlorpropandiol-(2,3) in alkalischem Medium umsetzt.

## Claims

1. 1,4-diamino-5-chloro-2-nitrobenzene derivatives to the general formula I

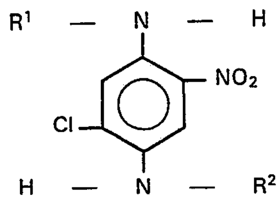

(I),

in which $R^1$ and $R^2$ can be the same or different and mean hydrogen, 2-hydroxyethyl or 2,3-dihydroxypropyl, with the proviso that at least one of the radicals $R^1$ and $R^2$ represents 2,3-dihydroxypropyl.

2. 1-amino-4-(2',3'-dihydroxypropyl)-amino-5--chloro-2-nitrobenzene.

3. 4-amino-1-(2',3'-dihydroxypropyl)-amino-5--chloro-2-nitrobenzene.

4. 1-(2'-hydroxyethyl)-amino-4-(2',3'-dihydroxypropyl-amino-5-chloro-2-nitrobenzene.

5. 1,4-di-[(2',3'-dihydroxypropyl)-amino]-5-chloro-2-nitrobenzene.

6. Means of colouring hair with a dyestuff content and conventional cosmetic additives, characterized in that it contains at least one compound according to Claim 1.

7. Means according to Claim 6 with a pH value of 8.0 to 11.5, adjusted by ammonia or organic amines such as monoethanolamine or triethanolamine.

8. Means for the oxidative colouring of hair, according to Claims 6 and 7, additionally containing at least one of the known oxydation hair dyes.

9. Means in the form of a hair dye with additional hair setting means according to Claim 6, additionally containing in acqueous-alcohol solution, known dyestuffs for direct application to the hair and at least one conventional cosmetic polymer.

10. A method of producing the compound according to Claim 1, characterized in that 1,4-diamino-5-chloro-2-nitrobenzene, possibly substituted on one of the amino groups with a 2-hydroxyethyl group, is reacted with chloropropanediol-(2,3) in an alkaline medium.

## Revendications

1. Dérivés 1,4-diamino-5-chloro-2-nitrobenzénique, répondant à la formule générale I

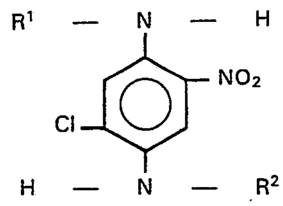

(I),

dans laquelle $R^1$ et $R^2$ peuvent être identiques ou différents et représentent l'hydrogène, le 2-hydroxyéthyle ou le 2,3-di-hydroxypropyle, sous la condition que l'un au moins des restes $R^1$ ou $R^2$ représente le 2,3-dihydroxypropyle.

2. 1-amino-4-(2',3'-dihydroxypropyl)-amino-5--chloro-2-nitrobenzène.

3. 4-amino-1-(2',3'-dihydroxypropyl)-amino-5--chloro-2-nitrobenzène.

4. 1-(2'-hydroxyéthyl)-amino-4-(2',3'-dihydroxypropyl)-amino-5-chloro-2-nitrobenzène.

5. 1,4-di-[(2',3'-dihydroxypropyl)-amido]-5-chloro-2-nitrobenzène.

6. Produit de coloration des cheveux renfermant des colorants et comportant des additifs cosmétiques usuels, caractérisé en ce qu'il renferme au moins un composé selon la revendication 1.

7. Produit selon la revendication 6 présentant une valeur de pH de 8,0 à 11,5 qui a été ajusté par de l'ammoniaque ou des amines organiques telles que la monoéthanolamine ou la triéthanolamine.

8. Produit de coloration des cheveux par voie d'oxydation selon les revendications 6 et 7, caractérisé en ce qu'il renferme, en outre, l'un au moins des colorants d'oxydation capillaires connus.

9. Produit sous forme de colorant pour cheveux assurant en outre la fixation des cheveux, selon la revendication 6, renfermant en outre, en solution dans un mélange eau-alcool, des colorants à montée directe sur les cheveux et au moins un produit de polymérisation cosmétique usuel.

10. Procédé de préparation du composé selon la revendication 1, caractérisé en ce que l'on fait réagir du 1,4-diamino-5-chloro-2-nitrobenzène, substitué éventuellement sur l'un des groupes amino par un groupe 2-hydroxyéthyle, sur du chloropropane-diol-(2,3), en milieu alcalin.